# EUROPEAN PATENT APPLICATION

(11) **EP 4 411 655 A1**
(43) Date of publication of application: **07.08.2024**
(21) Application number: 22874710.1
(22) Date of filing: 20.09.2022
(51) Int. Cl.: G06T 17/00, A61C 19/04, A61B 5/00

(54) **SCANNER AND SCANNING METHOD**

(30) Priority: 28.09.2021 CN 202111144855
(71) Applicant: Shining 3D Tech Co., Ltd., Hangzhou, Zhejiang 311258 (CN)
(72) Inventor: CHEN, Xiaojun, Hangzhou, Zhejiang 311258 (CN); ZHAO, Xiaobo, Hangzhou, Zhejiang 311258 (CN); JIANG, Tengfei, Hangzhou, Zhejiang 311258 (CN); QIAN, Wei, Hangzhou, Zhejiang 311258 (CN); FU, Xiaopeng, Hangzhou, Zhejiang 311258 (CN)
(74) Representative: Grünecker Patent- und Rechtsanwälte PartG mbB
(86) International application number: PCT/CN2022/119956
(87) International publication number: WO 2023/051323

(57) **Abstract**

The present disclosure relates to a scanner and a method for scanning. The scanner includes a projection optical system, a first acquisition optical system, a second acquisition optical system, and a data processing system. The projection optical system is configured to synchronously project visible light and infrared light with a stripe pattern to a target area. The first acquisition optical system is configured to acquire a first image based on the infrared light, where the first image is an image with the stripe pattern, and the first image includes facial information and tooth information. The second acquisition optical system is configured to acquire a second image based on the visible light, where the second image is a color texture image. The data processing system is configured to perform data processing based on the first image and the second image to obtain color three-dimensional model data of a measured object. Thereby, a new scanner and a method for scanning are provided, which acquire the first image including the facial information and the tooth information based on the infrared light with the stripe pattern. Based on this, the second image can be directly used to color the first image to achieve color scanning.

## Description

### Cross-Reference to Related Application

The present disclosure claims priority to Chinese Patent Application 202111144855.8, entitled "Scanner and Method for Scanning" filed with the Patent Office of China on September 28, 2021, which is incorporated by reference in the present disclosure.

### Technical Field

The present disclosure relates to the technical field of scanning technology, such as a scanner and a method for scanning.

### Background

Digital Smile Design (DSD) is a dental technique that integrates dental aesthetics into oral treatment, including the process of combining oral scanning data and facial data.

The scanning solution used for DSD can be a 3D solution, which requires aligning the oral scanning data and the facial data obtained by face scanning to the same coordinate system. In the process of coordinate alignment, there are generally three kinds of data. These three kinds of data include not only the oral scanning data and the facial data, but also stitching data, in which the oral scanning data can be obtained by an intraoral scanner, the facial data can be obtained by a facial scanner, and the stitching data can be obtained by one of the above intraoral scanner and facial scanner for performing stitching of the oral scanning data and the facial data.

In related 3D solutions, methods for obtaining the stitching data can include a visible light mode, which can obtain relatively clear facial data and partial tooth data (used as the stitching data) in the visible light mode.

### Summary

### (I) Technical problems to be solved

The technical problem to be solved by the present disclosure is to solve the existing problem of visible light flicker during a scanning process, which causes significant irritation to user's eyes, causing discomfort to user's eyes during the scanning, and resulting in poor user experience.

### (II) Technical solution

The following is a general description of the subject matter described in detail in the present disclosure, and the general description is not intended to limit the scope of protection of the claims.

The present disclosure provides a scanner and a method for scanning.

The present disclosure provides a scanner, which includes a projection optical system, a first acquisition optical system, a second acquisition optical system, and a data processing system;

the projection optical system is configured to synchronously project visible light and infrared light with a stripe pattern to a target area;

the first acquisition optical system is configured to acquire a first image based on the infrared light, where the first image is an image with the stripe pattern, and the first image includes facial information and tooth information;

the second acquisition optical system is configured to acquire a second image based on the visible light, where the second image is a color texture image; and

the data processing system is configured to perform data processing based on the first image and the second image to obtain color three-dimensional model data of a measured object.

In some embodiments of the present disclosure, the projection optical system includes a three-dimensional reconstruction projection optical system and a texture light source projection optical system;
the scanner further includes a control system, the control system is configured to synchronously trigger the three-dimensional reconstruction projection optical system and the texture light source projection optical system, so that the projection optical system synchronously projects the visible light and the infrared light to the target area; and
the three-dimensional reconstruction projection optical system is configured to project the infrared light to the target area, and the texture light source projection optical system is configured to project the visible light to the target area.

In some embodiments of the present disclosure, the first acquisition optical system includes a monochrome camera and a first filter component, and the first filter component is configured to filter out light of a preset waveband and only allow the infrared light to enter the monochrome camera;
and/or
the second acquisition optical system includes a color camera and a second filter component, and the second filter component is configured to cut off the infrared light and allow the visible light to enter the color camera.

In some embodiments of the present disclosure, the scanner further includes a support plate and support upright posts arranged on both sides of the support plate; and
the three-dimensional reconstruction projection optical system, the first acquisition optical system, and the second acquisition optical system are all fixed to the support plate, and the texture light source projection optical system is fixed to the support upright posts.

In some embodiments of the present disclosure, the scanner further includes a heat dissipation system; and
the heat dissipation system is configured to dissipate heat at least for the three-dimensional reconstruction projection optical system.

In some embodiments of the present disclosure, the texture light source projection optical system includes an LED array and a uniform light plate; and
the uniform light plate is arranged on a light emitting side of the LED array, and the uniform light plate is configured to uniform the light emitted by the LED array and project the light towards the target area.

In some embodiments of the present disclosure, the three-dimensional reconstruction projection optical system includes an infrared laser, a transmission plate with a stripe pattern, and an imaging lens; and after light emitted by the infrared laser passes through the transmission plate, the infrared light with the stripe pattern is projected by the imaging lens to the target area;
or the three-dimensional reconstruction projection optical system includes a digital light processing projector.

In some embodiments of the present disclosure, the stripe pattern includes a plurality of bright and dark alternating stripes; and
a value of a stripe duty cycle ranges from 10% to 50%.

The present disclosure further provides a method for scanning, performed by applying any of the above scanners, and the method for scanning includes:
synchronously projecting, by the projection optical system, the visible light and the infrared light with the stripe pattern to the target area;
acquiring, by the first acquisition optical system, the first image, where the first image is an image with the stripe pattern, and the first image includes the facial information and the tooth information;
acquiring, by the second acquisition optical system, the second image, where the second image is a color texture image; and
performing, by the data processing system, data processing based on the first image and the second image to obtain color three-dimensional model data of a measured object.

In some embodiments of the present disclosure, the performing data processing based on the first image and the second image to obtain color three-dimensional model data of the measured object includes:
determining a stripe sequence based on the first image, and performing stitching and fusing to construct the color three-dimensional data of the measured object;
determining a texture map with color texture for the measured object based on the second image; and
mapping the texture map onto the three-dimensional data to determine the measured object.

After reading and understanding accompanying drawings and detailed descriptions, other aspects can be understood.

### (III) Beneficial effects

Compared with the related art, the technical solution provided by the embodiment of the present disclosure has the following advantages:
In the scanner and the method for scanning provided by the embodiment of the present disclosure, the scanner includes the projection optical system, the first acquisition optical system, the second acquisition optical system, and the data processing system; the projection optical system is configured to synchronously project the visible light and the infrared light with the stripe pattern to the target area; the first acquisition optical system is configured to acquire the first image based on the infrared light, where the first image is an image with the stripe pattern, and the first image includes the facial information and the tooth information; the second acquisition optical system is configured to acquire the second image based on the visible light, where the second image is the color texture image; and the data processing system is configured to perform data processing based on the first image and the second image to obtain color three-dimensional model data of a measured object. Therefore, by arranging the projection optical system to synchronously project the visible light and the infrared light to the target area, such as the user's facial area, it is not necessary to switch between the infrared light and the visible light during the scanning process, that is, both the visible light and the infrared light are always bright, thereby avoiding the problem of visible light flicker and reducing the irritation to the user's eyes, which is conducive to enhancing the user experience during the scanning process. Meanwhile, the infrared light is infrared light with a stripe pattern, which is projected to the target area. Correspondingly, the first acquisition optical system obtains the first image based on the infrared light with the stripe pattern. Because the infrared light is in the form of a stripe, which can still be extracted even if it is diffused on the tooth surface, so that the first image can include both facial information and tooth information, which is equivalent to being able to simultaneously acquire stitching data and facial data based on the infrared light. Moreover, the second acquisition optical system can acquire a color texture image based on the visible light. Based on this, in the data processing system, data processing is directly performed based on the first image and the second image, for example, including reconstruction, stitching, fusion and other steps, that is, color data can be supplemented to the first image, so as to obtain the color three-dimensional model data of the measured object and achieve scanning of the measured object.

It should be understood that the general description above and the following detailed description are only exemplary and illustrative, and cannot limit the disclosure.

### Brief Description of Figures

The accompanying drawings herein are incorporated in and constitute a part of the specification, illustrating embodiments consistent with the present disclosure, and explaining the principles of the present disclosure together with the specification.

In order to provide a clearer explanation of the embodiments of the present disclosure or technical solutions in the related art, the accompanying drawings required in the embodiments or description of the related art will be briefly introduced below. It is evident that for those of ordinary skill in the art, other drawings may also be obtained based on these accompanying drawings without any creative effort.
FIG. 1 is a schematic structural diagram of a scanner according to some embodiments of the present disclosure;
FIG. 2 is a schematic diagram of the arrangement of a stripe pattern according to some embodiments of the present disclosure;
FIG. 3 is a schematic structural diagram of another scanner according to some embodiments of the present disclosure;
FIG. 4 is a schematic structural diagram of another scanner according to some embodiments of the present disclosure;
FIG. 5 is a schematic structural diagram of a front view of the scanner shown in FIG. 4;
FIG. 6 is a schematic structural diagram of a top view of the scanner shown in FIG. 4;
FIG. 7 is a schematic structural diagram of yet another scanner according to some embodiments of the present disclosure; and
FIG. 8 is a schematic flowchart of a method for scanning according to some embodiments of the present disclosure.

In the figures, 10: scanner; 101: support plate; 102: support upright post; 110: projection optical system; 111: three-dimensional reconstruction projection optical system; 112: texture light source projection optical system; 120: first acquisition optical system; 121: monochrome camera; 122: first filter component; 130: second acquisition optical system; 131: color camera; 132: second filter component; 140: data processing system; 150: control system; and 160: heat dissipation system.

### Detailed Description

In order to make the understanding of the above objectives, features and advantages of the present disclosure clear, technical solutions of the present disclosure will be described below. It should be noted that embodiments of the present disclosure and features in the embodiments can be combined with each other in the case of no conflict.

Many specific details are set forth in the following description in order to fully understand the present disclosure, but the present disclosure may also be implemented in other ways different from those described herein. Definitely, embodiments of the specification are merely a part of the embodiments of the present discourse and not all the embodiments.

Embodiments of the present disclosure provide a scanner and a method for scanning for obtaining stitching data based on infrared light with a stripe pattern. Because the infrared light with the stripe pattern can still be extracted after being diffused by teeth, the first data including tooth information can thus be acquired, that is, obtaining facial data and the stitching data can be simplified into the same step, and the stitching data may be tooth data. Meanwhile, visible light and the infrared light are synchronously triggered, and it is not necessary to switch between the two, thereby avoiding visible light flicker, reducing irritation to the user's eyes, and enhancing user experience.

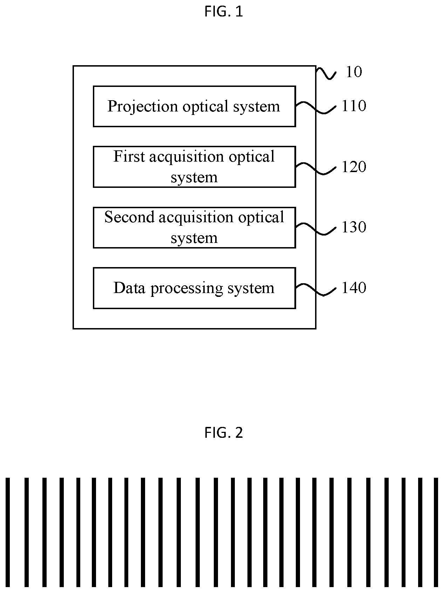

The following provides an exemplary explanation of the scanner and the method for scanning of embodiments of the present disclosure, in conjunction with FIG. 1 to FIG. 8.

In some embodiments of the present disclosure, a schematic structural diagram of a scanner according to some embodiments of the present disclosure is shown in FIG. 1. Referring to FIG. 1, the scanner 10 can include a projection optical system 110, a first acquisition optical system 120, a second acquisition optical system 130, and a data processing system 140. The projection optical system 110 is configured to synchronously project visible light and infrared light with a stripe pattern to a target area. The first acquisition optical system 120 is configured to acquire a first image based on the infrared light. The first image is an image with the stripe pattern. The first image includes facial information and tooth information. The second acquisition optical system 130 is configured to acquire a second image based on the visible light. The second image is a color texture image. The data processing system 140 is configured to perform data processing based on the first image and the second image to obtain color three-dimensional model data of a measured object.

The projection optical system 110 can synchronously project the visible light and the infrared light with the stripe pattern to the target area, without any switching between the visible light and the infrared light, thereby avoiding the irritation of visible light flicker to the user's eyes, reducing the discomfort to the user's eyes, and enhancing the user experience. Meanwhile, the target area can be the user's facial area, which can include the user's tooth area. The infrared light with the stripe pattern is projected to the user's face and partially to the user's tooth area, which is equivalent to linear projection of the infrared light. Therefore, even if the tooth will diffuse the infrared light, the diffused infrared light can still be extracted to obtain tooth information that can restore the teeth morphology, which is used as stitching data. Meanwhile, the facial information in the first image corresponds to facial data. Therefore, through subsequent processing of the first image and the second image, for example, including reconstruction, fusion, stitching and other steps, the second image is used to color the first image so as to achieve color scanning.

It can be understood that the target area may also be part or all of the area of other measured objects to be scanned, which is not limited here.

The first acquisition optical system 120 can acquire the first image based on the infrared light with the stripe pattern, and the first image includes the tooth information and the facial information, which can achieve synchronous acquisition of the stitching data and the facial data.

The second acquisition optical system 130 can acquire the second image based on the visible light, that is, the color texture image, which corresponds to texture data for coloring the first image.

The data processing system 140, which may also be referred to as a software algorithm system, can extract data based on the first image and the second image and perform data processing, for example, including reconstruction, fusion stitching, and texture mapping, so as to determine the color three-dimensional model data of the measured object, that is, to achieve the color scanning of the measured object.

In some embodiments of the present disclosure, the scanner 10 includes the projection optical system 110, the first acquisition optical system 120, the second acquisition optical system 130, and the data processing system 140. The projection optical system 110 synchronously projects visible light and infrared light to a target area, such as the user's facial area. Therefore, it is not necessary to switch between the infrared light and the visible light during the scanning process, that is, both the visible light and the infrared light are always bright, thereby avoiding the problem of visible light flicker and reducing the irritation to the user's eyes, which is conducive to enhancing the user experience during the scanning process. Meanwhile, the infrared light is infrared light with a stripe pattern, which is projected to the target area. Correspondingly, the first acquisition optical system 120 obtains the first image based on the infrared light with the stripe pattern. Because the infrared light is in the form of a stripe, which can still be extracted even if it is diffused on the tooth surface, so that the first image can include both facial information and tooth information, which is equivalent to being able to simultaneously acquire stitching data and facial data based on the infrared light. Moreover, the second acquisition optical system 130 can obtain a color texture image based on the visible light. Based on this, in the data processing system 140, data processing is directly performed based on the first image and the second image, that is, the stitching of texture data and the facial data can be achieved to obtain color three-dimensional model data of the measured object.

In some embodiments of the present disclosure, the stripe pattern includes a plurality of bright and dark alternating stripes; and a value of a stripe duty cycle ranges from 10% to 50%.

In some embodiments of the present disclosure, a value of a stripe line width ranges from 10 µm to 20 µm.

The stripe duty cycle can be understood as the ratio of the width of a bright stripe to the sum of the widths of adjacent bright and dark stripes, and the stripe line width is the line width of the bright stripe.

In this way, in a first aspect, the stripe pattern can be made into a coded pattern, so that a color three-dimensional reconstruction process based on the first image can be achieved to construct a color three-dimensional morphology of the measured object, for example, to construct the color three-dimensional morphology of the user's face. In a second aspect, by setting the value of the stripe line width ranging from 10 µm to 20 µm, in the stripe pattern projected to the user's face, the bright stripe can fall on the tooth without covering the entire individual tooth, and does not fall in the slit between teeth , so as to ensure that more accurate tooth information can be written in the first image ; and more accurate facial information can be carried to facilitate to achieve a more accurate color three-dimensional reconstruction process. In a third aspect, in conjunction with the setting of the stripe duty cycle, the specific value of the stripe duty cycle ranges from 10% to 50%, which can make the number of stripes more appropriate, so as to improve scanning efficiency while achieving accurate scanning.

Exemplarily, as shown in FIG. 2, the stripe pattern is composed of a plurality of equally spaced 101010101... stripes, the stripe duty cycle can be 20%, and the stripe line width can be 15 µm, where 0 represents black and 1 represents white. Exemplarily, the number of stripes in the stripe pattern may be 100 or other, which can be set based on the needs of the scanner.

Exemplarily, the stripe duty cycle can be 10%, 20%, 25%, 30%, 45%, 50% or other percentage values, and the stripe line width can be 10 µm, 15 µm, 18 µm, 20 µm or other width values which can be set based on the needs of the scanner, which is not limited here.

Exemplarily, the stripe duty cycle and the stripe line width can be fixed values built in the scanner, or can be adjustable values, for example, the user can identify user's input to achieve adjustment, or the scanner can achieve feedback adjustment based on the accuracy of scanning results, which is not limited here.

It can be understood that the stripe pattern can also be set to other types of coded patterns known to those skilled in the art, which is not limited here.

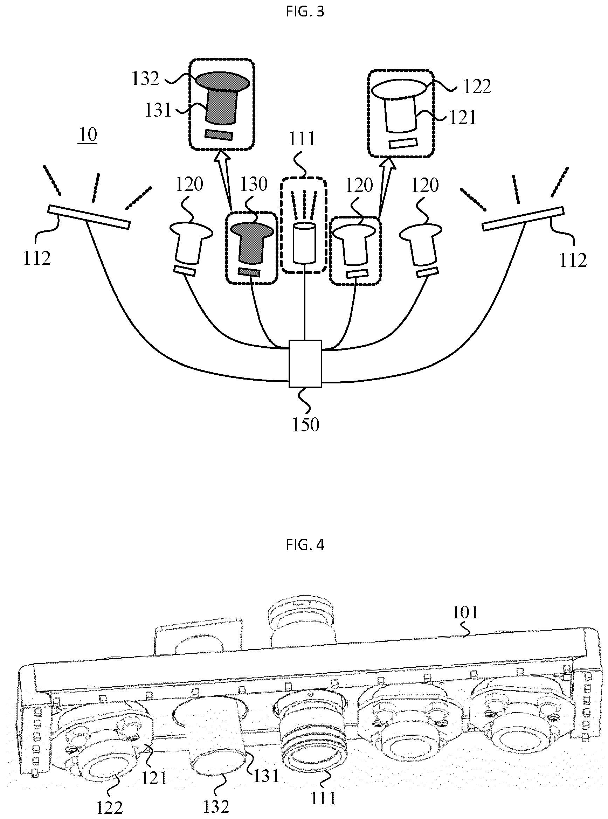

In some embodiments of the present disclosure, a schematic structural diagram of another scanner according to some embodiments of the present disclosure is shown in FIG. 3. In conjunction with FIG. 1 and FIG. 3, in the scanner 10, the projection optical system 110 includes a three-dimensional reconstruction projection optical system 111 and a texture light source projection optical system 112. The scanner 10 further includes a control system 150. The control system 150 is configured to synchronously trigger the three-dimensional reconstruction projection optical system 111 and the texture light source projection optical system 112, so that the projection optical system 110 synchronously projects the visible light and the infrared light to the target area. The three-dimensional reconstruction projection optical system 111 is configured to project the infrared light to the target area, and the texture light source projection optical system 112 is configured to project the visible light to the target area.

The control system 150 can synchronously trigger the three-dimensional reconstruction projection optical system 111 and the texture light source projection optical system 112 to enable the three-dimensional reconstruction projection optical system and the texture light source projection optical system to simultaneously project light, thereby achieving the effect that the projection optical system 110 synchronously projects the visible light and the infrared light with the stripe pattern to the target area.

Exemplarily, the control system 150 can be a timing control circuit, and output ends of the timing circuit are connected to the three-dimensional reconstruction projection optical system 111 and the texture light source projection optical system 112, respectively, to achieve synchronous triggering and synchronously project the infrared light and the visible light to the target area.

In some embodiments of the present disclosure, continuing to refer to FIG. 3, the first acquisition optical system 120 includes a monochrome camera 121 and a first filter component 122, and the first filter component 122 is configured to filter out light of a preset waveband and only allow the infrared light to enter the monochrome camera 121; and/or the second acquisition optical system 130 includes a color camera 131 and a second filter component 132, and the second filter component 132 is configured to cut off the infrared light and allow the visible light to enter the color camera 131.

The light of a preset waveband is light in other wavebands expect for the infrared light, and the first filter component 122 only allows the infrared light to enter the monochrome camera 121; meanwhile, the second filter component 132 is configured to cut off the infrared light, and the second filter component 132 can filter out the infrared light and the allow visible light to enter the color camera 131. Therefore, by arranging the first filter component 122 and the second filter component 132, the separation of the infrared light from the visible light is achieved, so that they enter the monochrome camera 121 and the color camera 131, respectively. Furthermore, based on this, it is equivalent to achieving the separation of the infrared light from the visible light in space, so that it is not necessary to separate the two in time, that is, it is not necessary to switch between the infrared light and the visible light, thereby avoiding visible light flicker, avoiding irritation to the user's eyes, and enhancing user experience.

Exemplarily, the number of the monochrome camera 121 can be three, which can also be referred to as three-dimensional cameras. The color camera 131 can also be referred to as a texture camera, and both the monochrome camera 121 and the color camera 131 can be Complementary Metal Oxide Semiconductor (CMOS) cameras, which is not limited here. The first filter component 122 can include a filter or other optics or optical components, and the second filter component 132 can be a cut-off filter or other optics or optical components, which is not limited here.

In some embodiments of the present disclosure, continuing to refer to FIG. 3, the control system 150 is also connected to the first acquisition optical system 120 and the second acquisition optical system 130. Therefore, a single frame of three-dimensional data only needs a trigger signal for one time, and the first image and the second image are acquired simultaneously. The first image corresponds to a reconstruction map, and the second image corresponds to a texture map. Therefore, there is no time difference between the reconstruction map and the texture map, which can meet conditions of color three-dimensional reconstruction with color texture required by a handheld three-dimensional scanner.

Exemplarily, the control system 150 triggers the projection optical system 110, so that the color three-dimensional reconstruction projection optical system 111 therein projects the infrared light with the stripe pattern to the measured object (face and teeth), while the texture light source projection optical system 112 projects the visible light to the measured object (face and teeth). Meanwhile, the control system 150 also controls the first acquisition optical system 120 and the second acquisition optical system 130 to acquire the second image based on the infrared light and the visible light, respectively. Exemplarily, a photosensitive waveband of the monochrome camera 121 corresponds to the waveband of the infrared light of the three-dimensional reconstruction projection optical system 111, and an infrared filter is arranged in front of a lens of the monochrome camera 121 to only allow the infrared light to pass through. Moreover, a photosensitive waveband of the color camera 131 corresponds to the waveband of the visible light of the texture light source projection optical system 112, and an infrared cut-off filter is arranged in front of a lens of the color camera 131 to filter out the infrared light and only allow the visible light to pass through. Therefore, the first acquisition optical system 120 and the second acquisition optical system 130 can quickly obtain an image of a measured object with a stripe pattern (i.e. the first image) and an image of the measured object with a color texture image (i.e. the second image) at the same time, respectively. The first image and the second image are respectively transmitted to the data processing system 140. The data processing system 140 performs data processing based on the first image and the second image to obtain the color three-dimensional model data of the measured object. The specific stitching process will be explained exemplarily in combination with methods hereinafter.

It can be understood that the monochrome camera 121 and the color camera 131 need to be subjected to position calibration before scanning to enable the texture map to correspond to the three-dimensional data. The specific position calibration way can be any one known to those skilled in the art, which is not repeated or limited here.

In some embodiments of the present disclosure, the three-dimensional reconstruction projection optical system 111 includes an infrared laser, a transmission plate with a stripe pattern, and an imaging lens. After light emitted by the infrared laser passes through the transmission plate, the infrared light with the stripe pattern is projected by the imaging lens to the target area.

The three-dimensional reconstruction projection optical system 111 can be used in the form of transmission projection, with the infrared laser as the light source, and the light emitted by the infrared laser passes through the stripe pattern and is projected out by the imaging lens, for example, to the target area. In this way, the three-dimensional reconstruction projection optical system 111 can project the infrared light with the stripe pattern to the target area.

In some embodiments of the present disclosure, the three-dimensional reconstruction projection optical system 111 includes a Digital Light Processing (DLP) projector.

In this way, an infrared DLP projector can also be used to project the infrared light with the stripe pattern.

In other embodiments of the present disclosure, the projection of the infrared light with the stripe pattern can also be achieved in other ways known to those skilled in the art, which is not limited here.

In some embodiments of the present disclosure, the texture light source projection optical system 112 includes an LED array and a uniform light plate. The uniform light plate is arranged on a light emitting side of the LED array, and the uniform light plate is configured to uniform the light emitted by the LED array and project the light towards the target area.

The texture light source projection optical system uses the LED array plus the uniform light plate to project white light (i.e. the visible light) to the facial area.

In other embodiments of the present disclosure, the visible light can be uniformly projected to the target area in other ways known to those skilled in the art, which is not limited here.

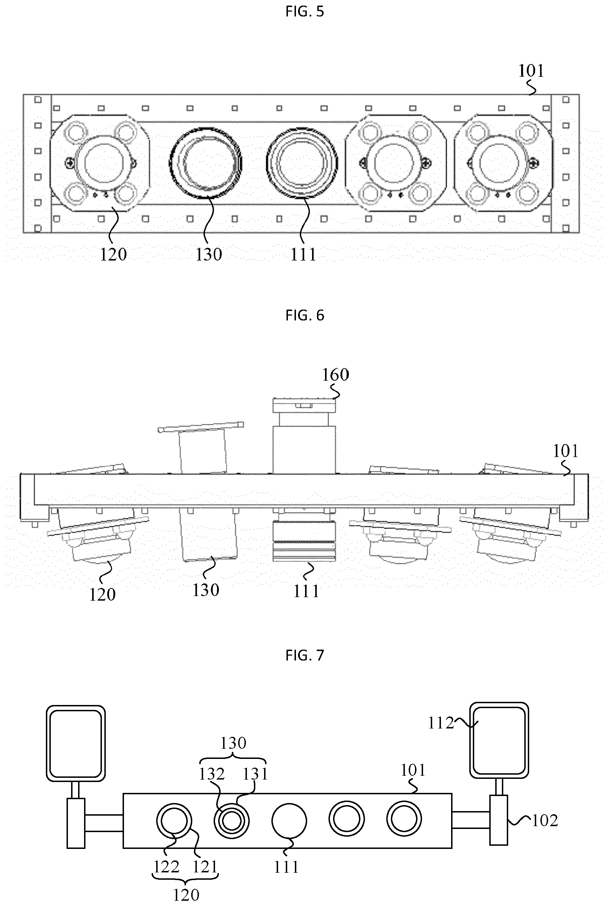

In some embodiments of the present disclosure, FIG. 4 is a schematic structural diagram of another scanner according to some embodiments of the present disclosure, FIG. 5 is a schematic structural diagram of a front view of the scanner shown in FIG. 4, and FIG. 6 is a schematic structural diagram of a top view of the scanner shown in FIG. 4. Referring to FIG. 4 to FIG. 6, the scanner 10 further includes a heat dissipation system 160. The heat dissipation system 160 is configured to dissipate heat at least for the three-dimensional reconstruction projection optical system 111.

When the scanner 10 works, the three-dimensional reconstruction projection optical system 111 produces more heat when projecting infrared light. By using the heat dissipation system 160 to dissipate heat therefrom, the three-dimensional reconstruction projection optical system 111 can work at an appropriate temperature, thereby ensuring that it has better performance, so as to ensure that the scanner has higher scanning accuracy.

Exemplarily, the heat dissipation system 160 is arranged on a back side of the three-dimensional reconstruction projection optical system 111, and it can be understood that the back side is a side that deviates from the light emitting side. In other embodiments of the present disclosure, the heat dissipation system 160 can also be arranged around the side of the three-dimensional reconstruction projection optical system 111, or arranged in other ways known to those skilled in the art, which is not limited here.

Exemplarily, the heat dissipation system 160 can include a heat dissipation fin structure arranged in an array, or the heat dissipation system 160 can use other heat dissipation structures known to those skilled in the art, which is not limited here.

In other embodiments of the present disclosure, the heat dissipation system 160 can also be used to dissipate heat for the control system 150, the first acquisition optical system 120, and the second acquisition optical system 130, or the heat dissipation system 160 can be used to dissipate heat for at least one of the three-dimensional reconstruction projection optical system 111, the texture light source projection optical system 112, the first acquisition optical system 120, the second acquisition optical system 130, the data processing system 140, and the control system 150, which can be arranged based on performance requirements and structural requirements of the scanner, which is not limited here.

In some embodiments of the present disclosure, FIG. 7 is a schematic structural diagram of yet another scanner according to some embodiments of the present disclosure. Referring to FIG. 7, the scanner 10 further includes a support plate 101 and support upright posts 102 arranged on both sides of the support plate 101. The three-dimensional reconstruction projection optical system 111, the first acquisition optical system 120, and the second acquisition optical system 130 are all fixed to the support plate 101, and the texture light source projection optical system 112 is fixed to the support upright posts 102.

The three-dimensional reconstruction projection optical system 111, the first acquisition optical system 120, and the second acquisition optical system 130 are all arranged on the support plate 101, and the texture light source projection optical system 112 is arranged on the support upright posts 102. On the one hand, reasonable arrangement of each structural component in the scanner 10 is achieved; on the other hand, it can make the structure of scanner 10 more compact while achieving better scanning effects, which is convenient for achieving miniaturization design of the scanner 10.

Exemplarily, in FIG. 7, it is only shown that the texture light source projection optical system 112 includes two overall square shaped illuminators to achieve illumination from opposite sides, thereby reducing shadow interference during unilateral illumination, and facilitating the second acquisition optical system 130 to accurately obtain the second image corresponding to the measured object in the target area. However, it does not constitute a limitation to the scanner 10 of the embodiment in the present disclosure. In other embodiments of the present disclosure, the number, shape, and spatial position of the illuminators in the texture light source projection optical system 112 can also be set in other ways known to those skilled in the art, which is not limited here.

Exemplarily, in conjunction with FIG. 3 and FIG. 7, the first acquisition optical system 120 can include 3 monochrome cameras 121, and each monochrome camera 121 is configured with a corresponding first filter component 122. The second acquisition optical system 130 can include 1 color camera 131, and the color camera 131 is configured with a corresponding second filter component 132. Moreover, 5 through holes are sequentially formed in the support plate 101 along the length direction, the three-dimensional reconstruction projection optical system 111 is fixed by the middle through hole, the color camera 131 is fixed by a through hole adjacent to the middle through hole, the monochrome camera 121 is fixed by the remaining three through holes in one-to-one correspondence, and the fixed angle of all monochrome cameras 121 can be arranged based on scanning requirements, which can meet the needs of three-dimensional reconstruction.

In other embodiments of the present disclosure, each structural component on the support plate 101 can also be arranged using other spatial layouts known to those skilled in the art, which is not limited here.

On the basis of the above embodiments, embodiments of the present disclosure further provide a method for scanning performed by applying any of the above scanners, which can achieve corresponding beneficial effects.

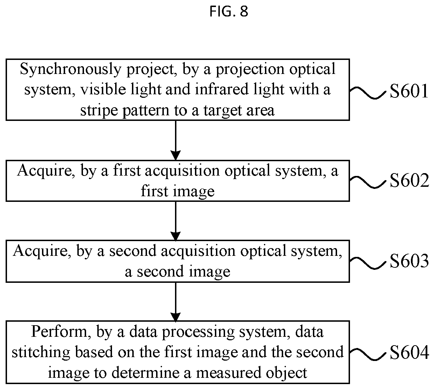

In some embodiments of the present disclosure, a schematic flowchart of a scanning method according to some embodiments of the present disclosure is shown in FIG. 8. Referring to FIG. 8, the method for scanning can include the following steps:
S601: Synchronously project, by the projection optical system, the visible light and the infrared light with the stripe pattern to the target area.

In conjunction with the above, the projection optical system can include a three-dimensional reconstruction projection optical system and a texture light source projection optical system, which can be synchronously triggered by the control system, so as to synchronously project the infrared light and the visible light to achieve the synchronous projection of the visible light and the infrared light by the projection optical system.

Exemplarily, the infrared light with the stripe pattern can also be referred to as infrared light with a coded pattern, which can be used in a subsequent three-dimensional reconstruction process to facilitate to achieve extraction of facial data and tooth data.

Exemplarily, the visible light can be white light, or other single waveband of visible light can be used, which can meet the needs of acquiring the second image, which is not limited here.

Exemplarily, taking the application of the scanner in the DSD scenario as an example, the target area can be an area including the user's facial area. In other scenarios, the target area can also be other areas that meet the needs for obtaining the measured object, which is not limited here.

S602: Acquire, by the first acquisition optical system, a first image.

The first image is an image with the stripe pattern, and the first image includes facial information and tooth information.

Therefore, corresponding to the DSD scenario, the first image can include facial data and stitching data.

S603: Acquire, by the second acquisition optical system, a second image.

The second image is a color texture image.

Therefore, corresponding to the DSD scenario, the second image can include texture data.

S604: Perform, by the data processing system, data processing based on the first image and the second image to obtain color three-dimensional model data of a measured object.

Therefore, by using the data processing based on the first image and the second image, stitching of the facial data and the texture data based on the stitching data can be achieved, thereby achieving the use of the second image to color the first image to determine the color three-dimensional model data of the measured object. For example, complete data of the user's face and teeth in the DSD scenario can be obtained.

In the method for scanning of the embodiment of the present disclosure, by arranging the projection optical system to synchronously project the visible light and the infrared light to the target area, such as the user's facial area, it is not necessary to switch between the infrared light and the visible light during the scanning process, that is, both the visible light and the infrared light are always bright, thereby avoiding the problem of visible light flicker and reducing the irritation to the user's eyes, which is conducive to enhancing the user experience during the scanning process. Meanwhile, the infrared light is infrared light with a stripe pattern, which is projected to the target area. Correspondingly, the first acquisition optical system obtains the first image based on the infrared light with the stripe pattern. Because the infrared light is in the form of a stripe, which can still be extracted even if it is diffused on the tooth surface, so that the first image can include both facial information and tooth information, which is equivalent to being able to simultaneously acquire stitching data and facial data based on the infrared light. Moreover, the second acquisition optical system can obtain a color texture image based on the visible light. Based on this, in the data processing system, data processing is directly performed based on the first image and the second image, that is, the stitching of texture data and the facial data can be achieved.

In some embodiments of the present disclosure, the performing data processing based on the first image and the second image to obtain color three-dimensional model data of a measured object includes:
determining a stripe sequence based on the first image, and performing stitching and fusing to construct the color three-dimensional data of the measured object;
determining a texture map with color texture for the measured object based on the second image; and
mapping the texture map onto the three-dimensional data to determine the color three-dimensional model data of the measured object.

In this embodiment of the present disclosure, based on a software algorithm built in the data processing system, on the one hand, the second image acquired by the second acquisition optical system (e.g., 1 CMOS color camera) is processed to obtain color three-dimensional data of the measured object with a color texture; on the other hand, the first image obtained by the first acquisition optical system (e.g., 3 CMOS monochrome cameras) is processed to determine a stripe sequence, and then the color three-dimensional data of the measured object is constructed by using a stripe three-dimensional reconstruction algorithm and a stitching fusion algorithm to determine the color three-dimensional morphology of the measured object; and finally, the texture map is mapped to the three-dimensional data by using a pre-calibrated position relationship between the monochrome cameras and the color cameras to determine the measured object.

It can be understood that the above algorithm can use any algorithm known to those skilled in the art to achieve the corresponding color three-dimensional reconstruction or stitching fusion process, which is not repeated or limited here. Meanwhile, the aforementioned way of pre-calibrating the position of the camera can be achieved using any position calibration way known to those skilled in the art, which is not limited here.

It should be noted that the "first", the "second" and other relational terms herein are used for distinguishing an entity or an operation from other entities and operations only, not necessarily to require or imply any actual relationship or sequence between the entities or the operations; and the terms, such as "comprise", "include" or any other variant, are intended to cover non-exclusive comprising, so that processes, methods, articles or devices containing a series of elements not only comprise the elements, but also comprise other elements which are not listed obviously, or comprise the inherent elements of the processes, the methods, the articles or the devices. The elements restrained by a statement "include a..." shall not exclude the condition that other same elements also exist in the processes, methods, articles or devices including the elements under the condition that no more restraints are required.

The foregoing are only specific implementations of the present disclosure so that those skilled in the art can understand or realize the present disclosure. Various modifications to the embodiments would be obvious for those skilled in the art, and a general principle defined herein can be implemented in other embodiments in the case of not departing from the spirit or scope of the present disclosure. Therefore, the present disclosure is not limited to the embodiments described herein, but should fall within a widest scope consistent with the principle and innovative characteristics disclosed herein.

### Industrial applicability

In the scanner and the method for scanning of the embodiments of the present disclosure, by arranging the projection optical system to synchronously project the visible light and the infrared light to the target area, such as the user's facial area, it is not necessary to switch between the infrared light and the visible light during the scanning process, that is, both the visible light and the infrared light are always bright, thereby avoiding the problem of visible light flicker and reducing the irritation to the user's eyes, which is conducive to enhancing the user experience during the scanning process. Meanwhile, the infrared light is infrared light with a stripe pattern, which is projected to the target area. Correspondingly, the first acquisition optical system obtains the first image based on the infrared light with the stripe pattern. Because the infrared light is in the form of a stripe, which can still be extracted even if it is diffused on the tooth surface, so that the first image can include both facial information and tooth information, which is equivalent to being able to simultaneously acquire stitching data and facial data based on the infrared light. Moreover, the second acquisition optical system can obtain a color texture image based on the visible light. Based on this, in the data processing system, data processing is directly performed based on the first image and the second image, for example, including reconstruction, stitching, fusion and other steps, that is, color data can be supplemented to the first image, so as to obtain the color three-dimensional model data of the measured object and achieve scanning of the measured object.

## Claims

1. A scanner, comprising a projection optical system, a first acquisition optical system, a second acquisition optical system, and a data processing system,
the projection optical system is configured to synchronously project visible light and infrared light with a stripe pattern to a target area;
the first acquisition optical system is configured to acquire a first image based on the infrared light, wherein the first image is an image with the stripe pattern, and the first image comprises facial information and tooth information;
the second acquisition optical system is configured to acquire a second image based on the visible light, wherein the second image is a color texture image; and
the data processing system is configured to perform data processing based on the first image and the second image to obtain color three-dimensional model data of a measured object.

2. The scanner as claimed in claim 1, wherein the projection optical system comprises a three-dimensional reconstruction projection optical system and a texture light source projection optical system;
the scanner further comprises a control system, the control system is configured to synchronously trigger the three-dimensional reconstruction projection optical system and the texture light source projection optical system, so that the projection optical system synchronously projects the visible light and the infrared light to the target area; and
the three-dimensional reconstruction projection optical system is configured to project the infrared light to the target area, and the texture light source projection optical system is configured to project the visible light to the target area.

3. The scanner as claimed in claim 2, wherein the first acquisition optical system comprises a monochrome camera and a first filter component, and the first filter component is configured to filter out light of a preset waveband and only allow the infrared light to enter the monochrome camera;
and/or
the second acquisition optical system comprises a color camera and a second filter component, and the second filter component is configured to cut off the infrared light and allow the visible light to enter the color camera.

4. The scanner as claimed in claim 2, further comprising a support plate and support upright posts arranged on both sides of the support plate; and
the three-dimensional reconstruction projection optical system, the first acquisition optical system, and the second acquisition optical system are all fixed to the support plate, and the texture light source projection optical system is fixed to the support upright posts.

5. The scanner as claimed in claim 2, further comprising a heat dissipation system, wherein,
the heat dissipation system is configured to dissipate heat at least for the three-dimensional reconstruction projection optical system.

6. The scanner as claimed in claim 2, wherein the texture light source projection optical system comprises an LED array and a uniform light plate; and
the uniform light plate is arranged on a light emitting side of the LED array, and the uniform light plate is configured to uniform the light emitted by the LED array and project the light towards the target area.

7. The scanner as claimed in claim 2, wherein the three-dimensional reconstruction projection optical system comprises an infrared laser, a transmission plate with a stripe pattern, and an imaging lens; and after light emitted by the infrared laser passes through the transmission plate, the infrared light with the stripe pattern is projected by the imaging lens to the target area;
or the three-dimensional reconstruction projection optical system comprises a digital light processing projector.

8. The scanner as claimed in any one of claims 1-7, wherein the stripe pattern comprises a plurality of bright and dark alternating stripes; and
a value of a stripe duty cycle ranges from 10% to 50%.

9. A method for scanning, performed by applying the scanner as claimed in any one of claims 1-8, comprising:
synchronously projecting, by the projection optical system, the visible light and the infrared light with the stripe pattern to the target area;
acquiring, by the first acquisition optical system, the first image, wherein the first image is an image with the stripe pattern, and the first image comprises the facial information and the tooth information;
acquiring, by the second acquisition optical system, the second image, wherein the second image is a color texture image; and
performing, by the data processing system, data processing based on the first image and the second image to obtain color three-dimensional model data of a measured object.

10. The method for scanning as claimed in claim 9, wherein the performing data processing based on the first image and the second image to obtain color three-dimensional model data of the measured object comprises:
determining a stripe sequence based on the first image, and performing stitching and fusing to construct the color three-dimensional data of the measured object;
determining a texture map with color texture for the measured object based on the second image; and
mapping the texture map onto the three-dimensional data to determine the color three-dimensional model data of the measured object.
